# EUROPÄISCHE PATENTSCHRIFT

(11) **EP 1 663 353 B1**
(45) Veröffentlichungstag und Bekanntmachung des Hinweises auf die Patenterteilung: **31.12.2008**
(21) Anmeldenummer: 04761904.4
(22) Anmeldetag: 08.09.2004
(51) Int. Cl.: A61M 5/20

(54) **VERABREICHUNGSVORRICHTUNG F R EIN INJIZIERBARES PRODUKT MIT EINER AUSL SESICHERUNG**
DEVICE FOR ADMINISTERING AN INJECTABLE PRODUCT, COMPRISING AN ANTI-RELEASE SAFETY DEVICE
DISPOSITIF D'ADMINISTRATION POUR PRODUIT INJECTABLE EQUIPE D'UNE SECURITE ANTI-DECLENCHEMENT

(30) Priorität: 11.09.2003 DE 10342058
(43) Veröffentlichungstag der Anmeldung: 07.06.2006
(73) Patentinhaber: TecPharma Licensing AG, 3401 Burgdorf (CH)
(72) Erfinder: SCHERER, Benjamin, CH-8610 Uster (CH)
(86) Internationale Anmeldenummer: PCT/CH2004/000564
(87) Internationale Veröffentlichungsnummer: WO 2005/023341

(56) Entgegenhaltungen:
- WO-A-99/37343
- CH-A- 518 102
- US-A- 5 167 632
- US-A- 6 099 503
- US-A- 6 135 979
- US-A- 6 159 181

## Beschreibung

Die vorliegende Erfindung betrifft eine Vorrichtung zur Verabreichung eines injizierbaren Produkts, insbesondere eine Autoinjektionsvorrichtung, mit einer Antriebseinrichtung zum Antrieb eines Antriebsglieds, einem Auslöser zum Auslösen der Antriebseinrichtung und einer Sicherungseinrichtung, die in einer Sicherungsposition eine Betätigung des Auslösers verhindert.

Es sind zahlreiche Modelle von Verabreichungsvorrichtungen für injizierbare Produkte, meist fluide Produkte, wie z. B. Insulin oder Wachstumshormone, bekannt. Das injizierbare Produkt wird z. B. in einer Ampulle innerhalb einem Gehäuse der Vorrichtung oder einer Kammer in dem Gehäuse untergebracht und kann durch einen Ausschüttmechanismus aus der Vorrichtung verabreicht werden. Zur Vereinfachung des Verabreichungsvorgangs für einen Arzt oder bei einer Selbstverabreichung durch einen Patienten, werden z. B. Autoinjektionsvorrichtungen verwendet, bei welchen eine in dem Gehäuse der Vorrichtung untergebrachte Injektionsnadel mittels eines Automatismus in eine Oberfläche, wie z. B. menschliches Gewebe, eingestochen wird und sofort anschließend ebenfalls automatisch das injizierbare Produkt durch eine Ausschütteinrichtung verabreicht wird. Ferner sind nadellose Verabreichungsvorrichtungen bekannt, bei welchen durch einen ausgewählten Druckverlauf das injizierbare Produkt derart aus der Vorrichtung austritt, dass es in eine Gewebeoberfläche penetriert und dann in das Gewebe verabreicht werden kann.

Um eine ungewollte oder versehentliche Auslösung einer Verabreichungsvorrichtung zu verhindern, sind verschiedene Sicherungseinrichtungen bekannt, die ein Auslösen der Verabreichungsvorrichtung verhindern. Aus der US 6,135,979 ist beispielsweise ein Verabreichungsgerät mit einer Medikamentenpatrone für ein fluides Produkt und einer komprimierten Gasfeder als Antriebseinrichtung für die Verabreichung des Produkts bekannt. Die Medikamentenpatrone und die Gasfeder sind in einem Gehäuse untergebracht, das aus einem ersten Gehäuseteil mit der Patrone, einem Auslass für das Produkt und einer Verschlusskappe und einem zweiten Gehäuseteil mit der Gasfeder besteht, wobei das zweite Gehäuseteil in Richtung der Verabreichung des Produkts auf das erste Gehäuseteil zu beweglich ist. Eine Antriebsstange schließt sich an die Gasfeder an und wird von einem Stift, der in eine Nut in der Stange eingreift, in einem vorgespannten gesicherten Zustand gehalten. In gesichertem Zustand sind die beiden Gehäuseteile in einem Abstand zueinander angeordnet und der Stift stößt an eine Innenseite des zweiten Gehäuseteils, so dass er die Stange nicht freigeben kann. Zur Sicherung werden die beiden Gehäuseteile durch ein Band in ihrem Abstand gehalten, das in dem Zwischenraum zwischen den Gehäuseteilen angeordnet ist. Zur Verabreichung des injizierbaren Produkts wird das Band abgerissen, so dass das zweite Gehäuseteil entlang der Längsachse der Vorrichtung in Verabreichungsrichtung bewegt werden kann, wenn das Verabreichungsgerät z. B. auf eine Gewebeoberfläche gepresst wird. Dabei bewegt sich der zweite Gehäuseteil gegenüber der Gasfeder, der Stange und dem Stift, so dass eine Öffnung in dem zweiten Gehäuseteil über dem Stift zu liegen kommt und dieser durch den Druck der Gasfeder aus der Nut der Stange bewegt wird. Dadurch wird die Stange freigegeben und kann durch den Gasdruck auf einen Kolben in der Medikamentenpatrone auftreffen und den Druck an diesen weitergeben, so dass das Produkt aus der Patrone verabreicht wird.

In der US 6,099,503 ist ein Autoinjektor beschrieben, in welchen eine Spritze einsetzbar ist. Als Antrieb ist in dem Autoinjektor eine Feder vorgesehen, welche die Spritze zum Einstechen der Nadel und den Kolben zum Ausschütten des Produkts antreibt. Die Feder wird in einer gespannten Position gehalten, in dem ein Auslöser eine Bewegung eines Antriebskolbens des Injektors, bzw. der Spritze, blockiert. Der Auslöser ist in der Blockierposition vorgespannt und wird durch eine Kontrollhülse in der Blockierposition gehalten. Durch Verschieben der Kontrollhülse wird der Auslöser entsichert und gibt automatisch auf Grund seiner Vorspannung die Antriebsfeder frei. Demnach erfolgt ein automatisches Einstechen und Ausschütten sobald die Kontrollhülse aus ihrer Sicherungsposition verschoben ist. Einem Anwender bleibt keine Möglichkeit, die Kontrollhülse zurück in ihre Sicherungsposition zu schieben, ohne dass der Autoinjektor ausgelöst wird.

Bei den bekannten Sicherungssystemen für Verabreichungsvorrichtungen ist es oftmals nicht möglich die Vorrichtung zu entsichern und sie wieder in einen gesicherten Zustand zurückzubringen, ohne dass eine Verabreichung oder eine andere Bewegung bei der Vorrichtung erfolgt ist. Ferner ist es bei den meisten Verabreichungsvorrichtungen vorgesehen, das Lösen der Entsicherung oder die anschliessende Verabreichung durch eine Bewegung in der Verabreichungsrichtung des Produkts durchzuführen. Der Anwender hat mit einer solchen Bewegungsrichtung in Verabreichungsrichtung die unmittelbare Assoziation, dass eine Verabreichung direkt folgt. Ausserdem ist es bei bekannten Verabreichungsvorrichtungen oft unvermeidlich, zu dem ohnehin zum Halten der Vorrichtung erforderlichen Druck auf die Gewebeoberfläche einen Auslösedruck auszuüben.

Für Patienten, die eine Verabreichung als äußerst unangenehm empfinden, ist es vorteilhaft, die Entsicherung und Verabreichung durch Bewegungen auszubilden, die nicht in der Verabreichungsrichtung erfolgen. Ein zusätzlicher Druck auf die Gewebeoberfläche kann dadurch vermieden werden. Ferner ist es vorteilhaft, wenn eine entsicherte Verabreichungsvorrichtung wieder in einen gesicherten Zustand gebracht werden kann, ohne dass eine Verabreichung erfolgen musste, z. B. wenn ein falscher Zeitpunkt für die Verabreichung gewählt wurde.

Es ist eine Aufgabe der vorliegenden Erfindung, eine Vorrichtung zur Verabreichung eines injizierbaren Produkts zu schaffen, die eine zuverlässige und leicht zu bedienende Sicherungseinrichtung bereitstellt, bei der jederzeit ein gesicherter oder entsicherter Zustand hergestellt werden kann, die eine Verabreichung erleichtert und ein einfaches Auslösen ermöglicht.

Die Aufgabe wird erfindungsgemäß durch eine Vorrichtung zur Verabreichung eines injizierbaren Produkts nach Anspruch 1 gelöst. Vorteilhafte Ausgestaltungen der Verabreichungsvorrichtung gehen aus den Unteransprüchen hervor.

Nach der vorliegenden Erfindung umfasst eine Vorrichtung zur Verabreichung eines injizierbaren Produkts eine Antriebseinrichtung zum Antrieb einer Bewegung eines Antriebsgliedes in Richtung einer Längsachse der Vorrichtung, einen Auslöser zum Auslösen der Antriebseinrichtung und eine Sicherungseinrichtung, die in einer Sicherungsposition eine Betätigung des Auslösers verhindert. Die Verabreichungsvorrichtung weist ein längliches Gehäuse auf, in dem die Antriebseinrichtung und ein Produktbehälter, wie z. B. eine Ampulle, untergebracht sind. Die Antriebseinrichtung kann z. B. von einem herkömmlichen Federmechanismus gebildet werden, bei dem eine Ausschütteinrichtung z. B. mit einer Kolbenstange von einer Spiralfeder in Richtung auf einen Kolben in der Ampulle hin angetrieben wird. Der Kolben drückt dann das fluide Produkt in der Ampulle auf einen gegenüberliegenden Auslass zu, so dass dieses ausgeschüttet wird. Die Erfindung kann aber auch bei einer Vorrichtung zur nadellosen Verabreichung verwendet werden. Ferner kann der Federmechanismus auch auf ein Vorschubelement einer Einstecheinrichtung zum Einstechen einer Injektionsnadel in eine Gewebeoberfläche wirken, so dass beim Auslösen der Antriebseinrichtung ein automatisches Einstechen der Injektionsnadel in die Oberfläche erfolgt. Natürlich sind auch andere Antriebsmechanismen als eine Spiralfeder für die Antriebseinrichtung denkbar.

Erfindungsgemäß befindet sich die Sicherungseinrichtung zur Sicherung der Betätigung des Auslösers vor einer Betätigung zunächst in einer Sicherungsposition und ist aus dieser entlang der Längsachse der Vorrichtung in eine Entsicherungsposition beweglich, in der der Auslöser betätigt werden kann. Vorzugsweise erfolgt die Bewegung zur Entsicherung der Verabreichungsvorrichtung entgegen einer Ausschüttrichtung, in der das Produkt aus der Verabreichungsvorrichtung ausgeschüttet wird, bzw. entgegen der Antriebsrichtung des Antriebsglieds. Ein Anwender hat dadurch beim Entsichern der Vorrichtung nicht die Assoziation, mit einer Entsicherung bereits kurz vor einer Verabreichung zu stehen.

Befindet sich die Sicherungseinrichtung in der Entsicherungsposition, wird der Auslöser von ihr derart freigegeben, dass er radial zur Längsachse beweglich ist. Der Auslöser kann dann manuell betätigt und die Antriebseinrichtung ausgelöst werden. Durch die radiale Betätigung des Auslösers, am besten durch seitliches Eindrücken in die Vorrichtung senkrecht auf die Längsachse zu, entsteht kein zusätzlicher Druck in Richtung der Längsachse der Verabreichungsvorrichtung.

In einer bevorzugten Ausführungsform der vorliegenden Erfindung wird der Auslöser durch eine langgestreckte Auslöserscheibe gebildet, die mit einem Ende seitlich aus der Verabreichungsvorrichtung herausragt, mit dem anderen Ende im Inneren der Vorrichtung liegt und in einem mittleren Bereich eine Öffnung mit einem schmalen und einem breiten Abschnitt aufweist. Das Antriebsglied der Antriebseinrichtung ist stabförmig in Längsrichtung der Verabreichungsvorrichtung ausgerichtet und weist an einem Ende einen Kopfbereich auf, der einen Bereich mit einem kleineren Durchmesser als an benachbarten Bereichen aufweist. Das Antriebsglied wirkt mit der Auslösescheibe derart zusammen, dass der Bereich mit dem kleinen Durchmesser formschlüssig in den schmalen Öffnungsabschnitt der Scheibe passt, so dass das Antriebsglied gegenüber der Auslösescheibe keine Translationsbewegung wie entlang der Längsachse der Vorrichtung ausführen kann. In einer Sicherungsposition wird demnach das Antriebsglied der Antriebseinrichtung von dem Auslöser in einer festen Position gegenüber dem Gehäuse der Verabreichungsvorrichtung gehalten. Die Antriebseinrichtung ist dann bereits vorgespannt.

Zum Auslösen der Antriebseinrichtung kann die Auslösescheibe in einer Entsicherungsposition der Sicherungseinrichtung radial zur Längsachse in das Gehäuse der Verabreichungsvorrichtung eingedrückt werden. Dabei verschiebt sich das Antriebsglied innerhalb der Öffnung in der Auslöserscheibe von dem schmalen Abschnitt zu dem breiten Abschnitt, da das Antriebsglied in radialer Richtung fest durch das Gehäuse der Verabreichungsvorrichtung gehalten wird. Der breite Abschnitt der Öffnung in der Auslösescheibe ist derart dimensioniert, dass der Bereich des Kopfbereichs des Antriebsgliedes mit großem Durchmesser durch diesen Abschnitt passt, so dass in dieser Stellung das Antriebsglied sich gegenüber der Auslöserscheibe in Längsrichtung bewegen kann. Beim Eindrücken der Auslöserscheibe in radialer Richtung wird daher das Antriebsglied der Antriebseinrichtung freigegeben und führt durch die Vorspannung der Antriebseinrichtung eine Bewegung in Richtung der Längsachse der Vorrichtung aus. Dadurch kann die Einstecheinrichtung zum Einstechen einer Injektionsnadel oder die Ausschütteinrichtung zur Ausschüttung des injizierbaren Produkts angetrieben werden. Vorteilhaft wird zunächst die Einstecheinrichtung angetrieben, die die Injektionsnadel in eine Gewebeoberfläche einsticht, und unmittelbar darauffolgend wird die Ausschütteinrichtung zur Ausschüttung des injizierbaren Produkts durch die Injektionsnadel in das Gewebe angetrieben.

Bei der bevorzugten Ausführungsform der Erfindung wird die Sicherungseinrichtung durch eine Hülse gebildet, die im Bereich der Auslöserscheibe um das Gehäuse der Verabreichungsvorrichtung angeordnet ist, vorzugsweise an einem Ende der Vorrichtung, das einer Nadeleinrichtung gegenüberliegt. Die Hülse kann auch einen Teil des Gehäuses bilden. Im Bereich der Auslöserscheibe weist die Hülse eine Öffnung auf, durch die die Scheibe hindurchtreten kann. Die Öffnung ist in Längsrichtung der Verabreichungsvorrichtung derart breit, dass die Hülse in Längsrichtung der Verabreichungsvorrichtung verschiebbar ist, trotz dem die Auslöserscheibe durch die Öffnung ragt. Die Auslöserscheibe weist an ihrem außen liegenden Ende einen Vorsprung auf, der senkrecht zur Scheibe und parallel zur Gehäusewandung verläuft. Zur Sicherung der Auslöserscheibe in der Position, in der die Scheibe das Antriebsglied fixiert, greift eine Hülsenwand in Längsrichtung, vorzugsweise in Verabreichungsrichtung, zwischen den Vorsprung und die Gehäusewandung. Durch das Eingreifen der Hülsenwand wird die Auslöserscheibe an einer Bewegung in radialer Richtung zur Längsachse gehindert, so dass die Sicherungseinrichtung blockiert ist und sich in einer Sicherungsposition befindet. Der Vorsprung kann auch durch eine Vertiefung in der Scheibe gebildet werden.

Zur Entsicherung der Verabreichungsvorrichtung wird die Hülse in Längsrichtung verschoben, bis die Hülsenwand aus dem Eingriff zwischen Scheibenvorsprung und Gehäusewandung gezogen ist und den Vorsprung der Auslöserscheibe freigibt. Die Hülsenöffnung stößt dann mit einer der Eingriffsseite gegenüberliegenden Seite gegen die Auslöserscheibe. Die Auslöserscheibe wird somit durch die Hülse nicht mehr an einer radialen Bewegung gehindert, so dass sie in radialer Richtung in das Gehäuse, bzw. die Hülse eingedrückt werden kann. Es ist jedoch auch möglich, die Hülse wieder in Gegenrichtung zu verschieben und die Hülsenwand wieder in Eingriff zwischen Scheibenvorsprung und Gehäusewandung zu bringen, so dass die Sicherungseinrichtung wieder in einer Sicherungsposition ist. Vorzugsweise wird die Hülse durch Führungseinrichtungen am Gehäuse in Bezug zum Gehäuse geführt. Weiter ist es vorteilhaft, eine Rastung vorzusehen, in der die Sicherungseinrichtung in der Sicherungsposition einrastet, so dass die Hülse zuverlässig in dieser Position gehalten wird. Zum Verschieben der Hülse ist dann ein geringer Anfangswiderstand zu überwinden, bis die Hülse in eine Entsicherungsposition verschoben werden kann.

Bei einer besonders bevorzugten Ausführungsform ist die Verabreichungsvorrichtung als wiederverwendbare Vorrichtung ausgebildet. Hierfür ist es möglich, die Antriebseinrichtung nach einer Verabreichung eines Produkts wieder in einen vorgespannten Zustand zurückzubringen und das Antriebsglied wieder durch den Auslöser zu fixieren. Die Rückstellung der Antriebseinrichtung kann z. B. beim Einsetzen einer neuen Ampulle erfolgen. Der Kopfbereich des Antriebsglieds wird dabei zurück durch die Öffnung der Auslöserscheibe geführt und die Auslöserscheibe wird z. B. durch eine Feder wieder in eine Anfangsposition gebracht, in der der Bereich mit dem kleinen Durchmesser des Kopfbereichs in den schmalen Abschnitt der Scheibenöffnung passt.

Bei einer anderen Ausführungsform der vorliegenden Erfindung wird von der Sicherungseinrichtung zusätzlich zur Sicherung des Auslösers auch ein Nadelschutz in einer Nadelschutzposition gesichert. Der Nadelschutz ist z. B. durch eine langgestreckte Hülse gegeben, die mit ihrem vorderen Ende in einer ersten vorgeschobenen Position die Injektionsnadel überragt, sich entlang der Längsachse der Vorrichtung bis zu dem Bereich erstreckt, an dem die Sicherungseinrichtung vorgesehen ist. In dem Sicherungszustand ist die Hülse der Sicherungseinrichtung in einem in Bezug auf die Verabreichungsrichtung vorgeschobenen, d. h. auf das Gehäuse aufgeschobenen Position. In dieser Position bildet sie einen Anschlag für die Nadelschutzhülse, so dass diese nicht entgegen der Verabreichungsrichtung entlang der Längsachse der Verabreichungsvorrichtung in eine zweite zurückgeschobene Position bewegt werden kann. Wird die Sicherungseinrichtung in eine Entsicherungsposition gebracht, d. h. entgegen der Verabreichungsrichtung entlang der Längsachse verschoben, entfernt sich der Anschlag von der Nadelschutzhülse. Die Nadelschutzhülse ist dann in der Entsicherungsposition um die gleiche Strecke wie die Sicherungshülse entgegen der Verabreichungsrichtung bewegbar und kann z. B. beim Aufsetzen auf eine Gewebeoberfläche in die zweite zurückgeschobene Position gebracht werden.

Zusätzlich ist es möglich, die Nadelschutzhülse mit einer weiteren Sicherungsfunktion für den Auslöser auszubilden. Hierfür wird die Nadelschutzhülse im Inneren der Sicherungshülse zumindest in dem Bereich fortgeführt, der dem in das Gehäuse mündenden Ende des Auslösers gegenüberliegt. Ist der Auslöser in einer Ausgangsposition, trifft er in einer Sicherungsposition der Nadelschutz- und der Sicherungshülse auf die Wand der Nadelhülse. Erst wenn die Sicherungshülse in eine Entsicherungsposition verschoben ist, ist die Nadelhülse verschiebbar und kann zurückgeschoben werden. In zurückgeschobenem Zustand der Nadelhülse liegt dem Auslöser eine Öffnung in der Nadelhülse gegenüber, durch die der Auslöser durchtreten kann, wenn er zum Auslösen der Antriebseinrichtung radial nach innen eingedrückt wird.

Eine Verabreichungsvorrichtung nach der vorliegenden Erfindung bildet einen größtmöglichen Schutz vor einer unbeabsichtigten Betätigung der Vorrichtung und vor Nadelstichverletzungen. Da sowohl die Sicherungseinrichtung als auch der Auslöser nicht in Richtung einer Verabreichung betätigt werden müssen, wird es einem Anwender erleichtert, die Hemmschwelle zum Auslösen einer Verabreichung zu überwinden. Zudem wird die Nadel zu jedem Zeitpunkt nicht nur vor Zugriff geschützt, sondern der weit über die Nadel herausragende Nadelschutz dient auch als Sichtschutz, so dass die Verabreichungsvorrichtung auch für Anwender mit einer Nadelphobie geeignet ist. Die Verabreichungsvorrichtung bietet eine einfache Handhabung, da nach der Entsicherung ein Druck auf den Auslöser ausreicht, um eine Verabreichung des Produkts ohne weitere Handgriffe durchzuführen.

Die Erfindung wird in Ausführungsbeispielen anhand der Zeichnung genauer beschrieben. In dieser stellen dar:
- Figur 1:: einen Längsschnitt durch eine Verabreichungsvorrichtung nach der vorliegenden Erfindung in einer ersten Ausführungsform in einer Sicherungsposition,
- Figur 2:: einen Längsschnitt einer Verabreichungsvorrichtung nach Figur 1 in einer Entsicherungsposition,
- Figur 3:: einen Längsschnitt einer Verabreichungsvorrichtung nach Figur 1 in einem ausgelösten Zustand,
- Figur 4:: einen Längsschnitt durch eine Verabreichungsvorrichtung nach Figur 1 nach einer Verabreichung,
- Figur 5:: einen Längsschnitt durch eine Verabreichungsvorrichtung nach der vorliegenden Erfindung in einer zweiten Ausführungsform in einer Sicherungsposition und
- Figur 6:: einen Längsschnitt einer Verabreichungsvorrichtung nach Figur 5 nach einer Verabreichung.

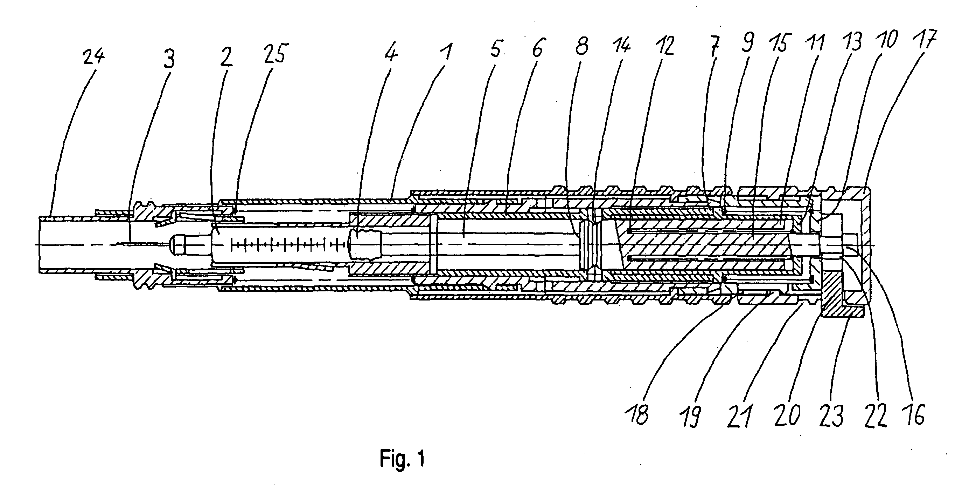

In Figur 1 ist eine Verabreichungsvorrichtung für ein injizierbares Produkt in Form eines Autoinjektionspens gezeigt. Der Injektionspen weist ein längliches Gehäuse 1 auf, in dem eine Ampulle 2 mit einem injizierbaren Produkt untergebracht ist, an die sich in Verabreichungsrichtung eine Injektionsnadel 3 auswechselbar anschließt. An einem der Injektionsnadel gegenüberliegenden Ende der Ampulle 2 ist in dieser ein Kolben 4 untergebracht, der die Ampulle 2 abschließt. An den Kolben 4 schließt sich eine Kolbenstange 5 an, die sich in Längsrichtung des Injektionspens erstreckt. Die Einheit aus Injektionsnadel 3, Ampulle 2, Kolben 4 und Kolbenstange 5 ist fest in einer Einstechhülse 6 gelagert, die gegenüber dem Gehäuse 1 in Längsrichtung relativ verschiebbar ist und in der in Figur 1 gezeigten, in das Gehäuse 1 eingefahrenen Position z. B. durch einen kleinen Rastwiderstand fixiert ist. Die Einstechhülse 6 ist über einen Anschlag 7 mit einer Antriebseinrichtung verbunden, durch die die Einstechhülse 6 in Verabreichungsrichtung, d. h. in der Richtung, in der die Injektionsnadel 3 weist, entlang der Längsachse des Injektionspens verschiebbar ist, so dass mittels der Antriebseinrichtung ein automatisches Einstechen der Injektionsnadel 3 in eine Gewebeoberfläche erfolgen kann.

Die Kolbenstange 5 ist ebenfalls an die Antriebseinrichtung über einen Anschlag 8 angeschlossen, um den Kolben 4 in Verabreichungsrichtung entlang der Längsachse innerhalb der Ampulle 4 für eine Verabreichung des injizierbaren Produkts verschieben zu können.

Die Antriebseinrichtung weist zum Antrieb der Einstechhülse 6 eine erste Feder 9 auf, die zwischen dem Anschlag 7 für die Einstechhülse 6 und einer hinteren senkrecht zur Längsachse des Injektionspens verlaufenden Gehäusewand 10 eingesetzt und in Figur 1 in einem vorgespannten komprimierten Zustand ist. Innerhalb der Einstechhülse 6 befindet sich eine Ausschütthülse 11, die sich an die Kolbenstange 5 anschließt und relativ zu der Einstechhülse 6 und dem Gehäuse 1 in Längsrichtung des Injektionspens verschiebbar ist. Zum Antrieb der Ausschütthülse 11 weist die Antriebseinrichtung eine zweite Feder 12 auf, die zwischen einer die Einstechhülse 6 abschließenden Wand 13 und einem Anschlag an der Ausschütthülse 11 eingesetzt ist. In Figur 1 ist die zweite Feder 12 in einem vorgespannten komprimierten Zustand. Die Ausschütthülse 11 wird innerhalb der Einstechhülse 6 z. B. durch einen Widerstand 14 in der vorgespannten Position gehalten, bis der Einstechvorgang abgeschlossen ist. Die dargestellte Einstech- und Ausschütteinrichtung ist Gegenstand einer parallelen Anmeldung der Anmelderin auf die hiermit Bezug genommen wird.

Die Antriebseinrichtung umfasst daher wenigstens die in Längsrichtung des Injektionspens verschiebbare Einstechhülse 6 und die Ausschütthülse 11 sowie die erste Feder 9 und die zweite Feder 12.

An der Ausschütthülse 11 ist eine stangenartige Verlängerung 15 vorgesehen, die vorzugsweise an einem vorderen Ende der Ausschütthülse 11 beginnt, sich entlang der Achse der Ausschütthülse 11 durch diese hindurch erstreckt und über das hintere Ende aus der Ausschütthülse 11 austritt. Das aus der Ausschütthülse 11 austretende Ende der Verlängerung 15 ragt durch eine Öffnung in der Wand 13 und eine Öffnung in der Wand 10 hindurch und weist an diesem Ende einen Kopfbereich 16 auf, der an einem Bereich einen kleineren Durchmesser als an den angrenzenden Bereichen hat.

Über dem Ende des Injektionspens, aus dem die Verlängerung 15 herausragt, ist als Sicherungseinrichtung eine Hülsenkappe 17 vorgesehen. Die Hülsenkappe 17 ist in Längsrichtung des Injektionspens verschiebbar auf diesem angeordnet und kann eine Art Gehäuseabschluss für den Injektionspen bilden. Zur Führung der Hülsenkappe 17 sind Führungseinrichtungen in Form von Führungsrillen 18 an dem Gehäuse 1 und nach innen gerichteten, in die Führungsrillen 18 eingreifenden Führungsvorsprünge 19 an der Hülsenkappe 17 vorgesehen. Die Führungseinrichtung kann gleichzeitig eine Rastung vorsehen, welche die Hülsenkappe 17 in der in Figur 1 gezeigten eingeschobenen Position zeigt, d. h. in einer in Verabreichungsrichtung vordersten Position.

Als Auslöser zum Auslösen der Antriebseinrichtung weist der Injektionspen eine länglich ausgebildete Auslöserscheibe 20 auf, die senkrecht zur Längsrichtung des Injektionspens angeordnet ist und durch eine Öffnung 21 in der Hülsenkappe 17 mit einem Ende seitlich aus dem Injektionspen heraustritt. Das andere Ende der Auslöserscheibe 20 mündet ins Innere des Gehäuses 1. In dem im Inneren des Pens liegenden Bereich der Auslöserscheibe weist diese eine Öffnung 22 mit einem schmalen Öffnungsabschnitt, dessen Breite dem kleinen Durchmesser des Kopfbereichs 16 der Verlängerung 15 entspricht und einem breiten Öffnungsabschnitt, dessen Breite dem größeren Durchmesser des Kopfbereichs 16 der Verlängerung entspricht.

Wie in Figur 1 gezeigt ist, ragt der Kopfbereich 16 der Verlängerung 15 durch die Öffnung 22 der Auslöserscheibe 20 hindurch, wobei der kleine Durchmesser des Kopfbereichs 16 in dem schmalen Öffnungsabschnitt der Öffnung 22 formschlüssig zu liegen kommt, so dass die an den Bereich mit kleinem Durchmesser angrenzenden Bereiche mit größerem Durchmesser des Kopfbereichs 16 einen Anschlag der Verlängerung 15 an der Auslöserscheibe 20 bilden und die Verlängerung 15 nicht innerhalb der Öffnung 22 verschiebbar ist. Die Antriebseinrichtung ist daher aufgrund der komprimierten Federn in einem durch die Auslöserscheibe 20 gehaltenen vorgespannten Zustand.

An dem seitlich aus dem Injektionspen herausragenden Ende weist die Auslöserscheibe 20 einen Vorsprung in Form einer Nase 23 auf, die senkrecht von der Auslöserscheibe 20 in Längsrichtung des Injektionspens entgegen der Verabreichungsrichtung abragt. Die Auslöserscheibe 20 tritt soweit aus der Hülsenkappe 17 durch die Öffnung 21 heraus, dass eine Innenwand der Nase 23 an einer Außenwand der Hülsenkappe 17 parallel zu liegen kommt. Der Abstand zwischen der Naseninnenwand, d. h. der dem Gehäuse zugewandten Nasenwand, und der zu dieser Naseninnenwand am weitesten entfernt liegenden Öffnungswand der Öffnung 22, die den schmalen Öffnungsabschnitt der Öffnung 22 abschließt, ist derart abgestimmt, dass die Auslöserscheibe 20 nicht in radialer Richtung zur Längsachse beweglich ist, solange die Naseninnenwand an der Außenwand der Hülsenkappe 17 an der Nasenwand anliegt. Die Auslöserscheibe 20 ist deshalb nicht in radialer Richtung verschiebbar und kann daher keine Relativbewegung gegenüber der Verlängerung 15 ausführen. In diesem in Figur 1 gezeigten Zustand befindet sich die Hülsenkappe 17 in einem Sicherungszustand für den Injektionspen und wird in diesem z. B. durch die Rastung in der Führungseinrichtung gehalten. Ein ungewolltes und versehentlichen Auslösen des Injektionspens wird dadurch verhindert.

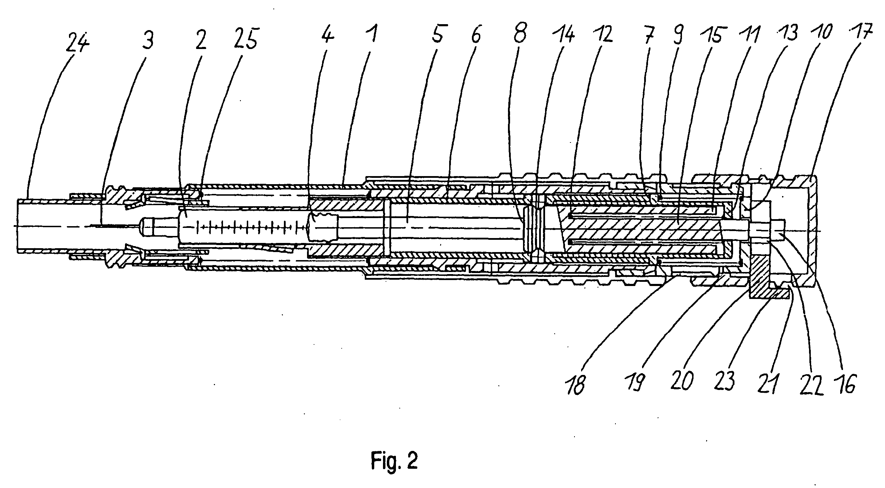

In Figur 2 ist der Injektionspen der Ausführungsform nach Figur 1 in einer Entsicherungsposition gezeigt. Zur Entsicherung des Injektionspens wurde die Hülsenkappe 17 von dem Injektionspen zurückgezogen, d. h. entgegen der Verabreichungsrichtung entlang der Längsachse des Pens innerhalb der Führungseinrichtung in eine hintere, am weitesten von der Injektionsnadel entfernte Position verschoben. Die Länge der Öffnung 21 der Hülsenkappe 17 in Längsrichtung entspricht der Länge der Nase 23, d. h. der Dicke der Auslöserscheibe 20 plus der Länge der abragenden Nase. Beim Abziehen der Hülsenkappe 17 zum Entsichern des Injektionspens kann die Kappe 17 solange entgegen der Verabreichungsrichtung auf dem Gehäuse 1 verschoben werden, bis die Öffnung 21 an die Auslöserscheibe 20 stößt, und zwar auf der der Nase 23 gegenüberliegenden Seite. Dabei wird die Wand der Hülsenkappe 17, die der Nase 23 gegenüberlag, mit verschoben. Der Nasenwand liegt daher nicht mehr ein Wandbereich der Hülsenkappe 17, sondern ein Öffnungsbereich der Öffnung 21 gegenüber. Die Sperrung der Radialbewegung der Auslöserscheibe 20 durch die Hülsenkappe 17, wie sie in Figur 1 dargestellt ist, ist in Figur 2 durch die herausgezogene Hülsenkappe 17 aufgehoben, so dass sich der Injektionspen in einer Entsicherungsposition befindet. Gemäß der vorliegenden Erfindung ist es vorteilhaft, dass die Hülsenkappe 17 auch ohne Betätigung der Auslöserscheibe 20 wieder in eine vordere Position gegenüber dem Gehäuse 1 vorgeschoben werden kann, in der sie wieder eine Bewegung der Auslöserscheibe 20 blockiert und damit wieder eine Sicherungsposition einnimmt.

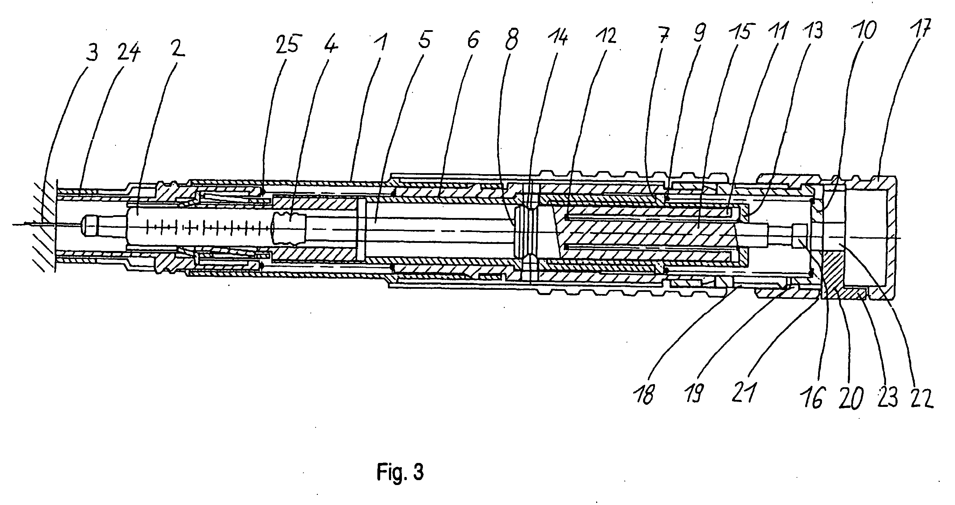

In Figur 3 ist der Injektionspen in einer ausgelösten Position gezeigt, in der die Auslöserscheibe 20 in radialer Richtung auf die Längsachse des Injektionspens zu bewegt wurde. Hierfür wird die Auslöserscheibe 20 manuell, z. B. mit dem Daumen einer Hand, in die Hülsenkappe 17 eingedrückt, wobei sich die Verlängerung 15 innerhalb der Öffnung 21 von dem schmalen Öffnungsabschnitt in den breiten Öffnungsabschnitt bewegt. Der breite Öffnungsabschnitt der Öffnung 21 ist derart breit, dass auch die Bereiche mit einem großen Durchmesser des Kopfbereichs 16 der Verlängerung 15 durch die Öffnung 21 passen und daher die Verlängerung innerhalb der Öffnung 21 beweglich ist und aus ihr austreten kann. Nach dem Eindrücken der Auslöserscheibe 20 kommt diese mit ihrer Nase 23 innerhalb der Öffnung 21 der Hülsenkappe 17 zu liegen. Die Hülsenkappe 17 ist dann nicht in eine vordere eingefahrene Position verschiebbar.

Durch das Eindrücken der Auslöserscheibe 20 wird die Antriebseinrichtung ausgelöst und es erfolgt ein Einstech- und Ausschüttvorgang aufgrund der vorgespannten Federn 9 und 12. Sobald das formschlüssige Zusammenwirken zwischen der Auslöserscheibe 20 und der Verlängerung 15 durch das Eindrücken der Auslöserscheibe aufgehoben wird, wird zunächst die Einstechhülse 6 durch die Kraft der ersten Feder 9 relativ zu dem Gehäuse 1 in Verabreichungsrichtung verschoben, so dass die Injektionsnadel 3 in Richtung der Gewebeoberfläche bewegt wird.

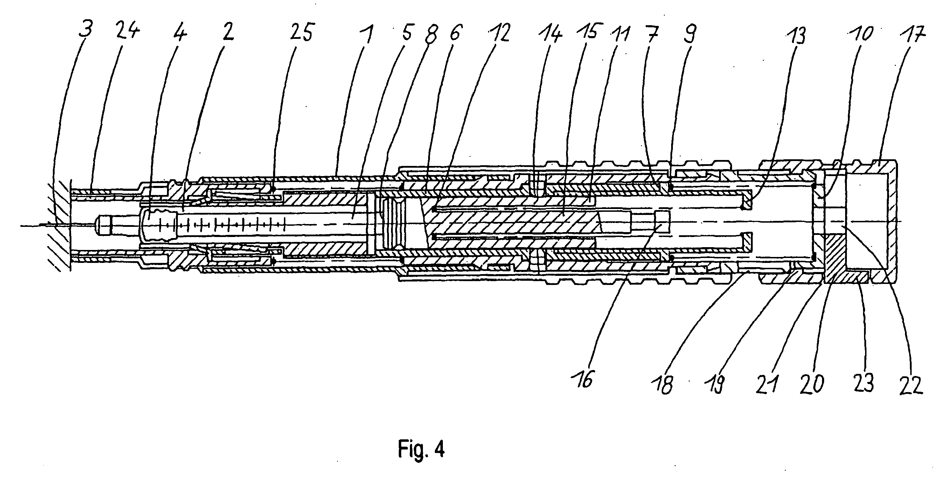

Anschließend wird die Ausschütthülse 11 aufgrund der Kraft der zweiten Feder 12 ebenfalls relativ zu dem Gehäuse 1 und der Einstechhülse 6 in Verabreichungsrichtung bewegt, so dass der Kolben 4 innerhalb der Ampulle 2 in Richtung der Injektionsnadel verschoben wird und dadurch eine Ausschüttung durch die eingestochene Nadel erfolgt, wie in Figur 4 gezeigt ist. Um bei der Verabreichung einen Ablauf sicherzustellen, nachdem zuerst die Injektionsnadel 3 in eine Gewebeoberfläche eingestochen wird, und dann dass Produkt aus der Ampulle 2 verabreicht wird, sind die Federn 9 und 12 und die zwischen dem Gehäuse 1, der Einstechhülse 6 und der Ausschütthülse 11 vorgesehenen Widerstände entsprechend aufeinander abgestimmt.

Wie in den Figuren 3 und 4 gezeigt ist, ist es bei der beschriebenen Ausführungsform möglich, eine gegenüber dem Gehäuse 1 verschiebbare Nadelschutzhülse 24 vorzusehen, die so weit von dem Gehäuse 1 in Verabreichungsrichtung hervorsteht, dass sie die Injektionsnadel 3 auch in einem vorgeschobenen Zustand umgibt. Zur Verabreichung des Produkts wird der Injektionspen mit der Nadelschutzhülse 24 senkrecht auf eine Gewebeoberfläche aufgedrückt und die Nadelschutzhülse 24 ins Innere des Gehäuses 1 zurück verschoben, bis ein Rand des Gehäuses 1 auf der Oberfläche zu liegen kommt. Beim Einstechen der Injektionsnadel 3 durch die Antriebseinrichtung wird die Injektionsnadel 3 über den Rand des Gehäuses 1 und der Nadelschutzhülse 24 hinaus in Verabreichungsrichtung in die Gewebeoberfläche eingestochen. Nach der Verabreichung wird der Injektionspen von der Gewebeoberfläche abgenommen und die Injektionsnadel 3 aus der Gewebeoberfläche herausgezogen. Gleichzeitig wird die Nadelschutzhülse 24 von einer Feder 25 in eine über das Gehäuse 1 hinausragende Stellung zurück verschoben. Die Feder 25 ist zwischen der Nadelschutzhülse 24 und einem Anschlag an dem Gehäuse 1 eingesetzt. Die Injektionsnadel 3 ist daher zu jedem Zeitpunkt durch die Nadelschutzhülse 24 geschützt, auch wenn sie innerhalb des Injektionspens in einer vorgeschobenen Position ist.

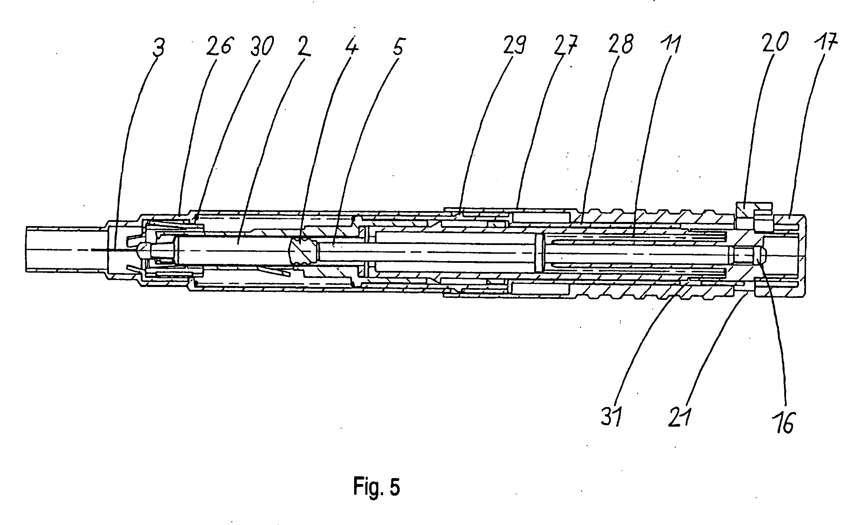

In Figur 5 ist eine zweite Ausführungsform einer Verabreichungsvorrichtung in Form eines Injektionspens nach der vorliegenden Erfindung dargestellt. Der Sicherungsmechanismus erfolgt in gleicher Weise, wie in der in den Figuren 1 bis 4 gezeigten Ausführungsform, durch die Hülsenkappe 17 und die Auslöserscheibe 20. Zusätzlich ist jedoch eine weitere Sicherungseinrichtung vorgesehen, durch die sichergestellt werden soll, dass eine Auslösung erst erfolgt, wenn ein Nadelschutz in einem eingefahrenen Zustand ist, d. h. der Injektionspen ordnungsgemäß auf eine Gewebeoberfläche aufgesetzt ist. Hierfür weist der Nadelschutz eine vordere Nadelhülse 26, die einen Teil des Gehäuses bildet und gegenüber einem Grundgehäuse 27 verschiebbar ist, und eine Sicherungshülse 28 auf, die gegenüber der Nadelhülse 26 und dem Grundgehäuse 27 verschiebbar ist. Die Hülsenkappe 17 ist gegenüber dem Grundgehäuse 27 zur Sicherung des Injektionspens verschiebbar. An der Sicherungshülse 28 sind Anschläge 29 vorgesehen, an die ein hinteres Ende der Nadelhülse 26 anschlägt, so dass die Nadelhülse 26 bei einer Bewegung relativ zu dem Grundgehäuse 27 entgegen der Verabreichungsrichtung die Sicherungshülse 28 bei dieser Bewegung mitnimmt. Dabei wird eine Feder 30 komprimiert. Die Sicherungshülse 28 ragt mit ihrem der Nadelhülse 26 gegenüberliegenden Ende durch das Grundgehäuse 27 hindurch und entlang der Innenseite in die Hülsenkappe 17 bis über den Bereich hinein, dem die Auslöserscheibe 20 gegenüberliegt. Das in die Hülsenkappe 17 mündende Ende der Auslöserscheibe 20 kommt gegenüber der Innenseite der Sicherungshülse 28 zu liegen, wenn der Injektionspen in einer gesicherten Position ist, d. h. die Nadelhülse 26 und die Sicherungshülse 28 in einer aus dem Grundgehäuse 27 herausgezogenen Position sind. Das in die Hülsenkappe 17 hineinragende Ende der Sicherungshülse 28 weist eine Öffnung 31 auf, deren Durchmesser der Breite der Auslöserscheibe 20 entspricht.

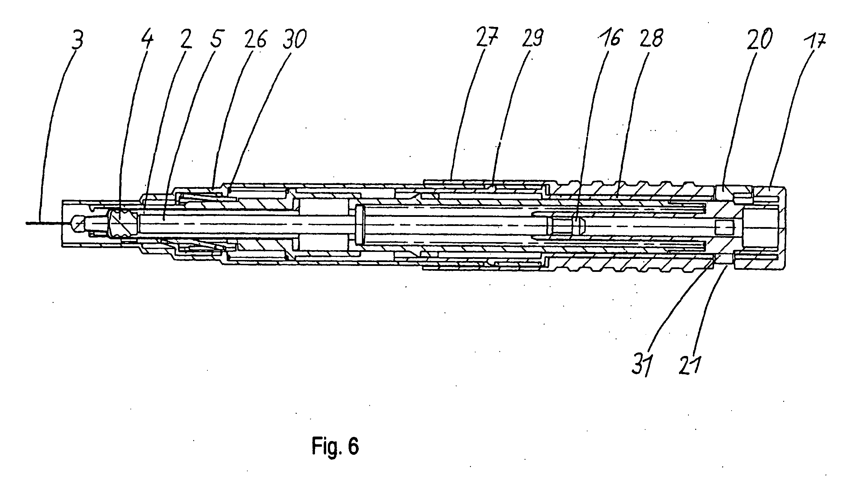

Wie in Figur 6 gezeigt ist, wird beim Aufsetzen des Injektionspens auf eine Gewebeoberfläche die Nadelhülse 26 und die Sicherungshülse 28 in das Grundgehäuse 27 hinein verschoben, bis die Öffnung 31 der Sicherungshülse 28 der Auslöserscheibe 20 gegenüberliegt. Nach dem Herausziehen der Hülsenkappe 17 kann, wie bei dem ersten Ausführungsbeispiel der Figuren 1 bis 4, die Auslöserscheibe 20 in radialer Richtung eingedrückt werden, wobei das in der Hülsenkappe 17 befindliche Ende der Auslöserscheibe 20 durch die Öffnung 31 der Sicherungshülse 28 hindurchtritt und die Verlängerung 15 aus der Öffnung 22 austritt. Dadurch wird die Antriebseinrichtung ausgelöst und es kann ein Einstechvorgang erfolgen, bei dem die Injektionsnadel 3 über den vorderen Rand der Nadelhülse 26 hinaus tritt.

Beim Absetzen des Injektionspens von der Gewebeoberfläche, d. h. beim Herausziehen der Injektionsnadel 3 aus der Gewebeoberfläche, wird die Nadelhülse 26 durch die Feder 30 wieder in Verabreichungsrichtung in eine vorgeschobene Position gebracht, in der sie die vorgehobene Injektionsnadel 3 umgibt. Die Sicherungshülse 28 verbleibt dabei gegenüber dem Grundgehäuse in Ruhe, so dass sich die Nadelhülse 26 auch gegenüber der Sicherungshülse 28 verschiebt.

Wie aus den beiden gezeigten Ausführungsbeispielen der vorliegenden Erfindung ersichtlich ist, sind verschiedene Modifikationen bei Verabreichungsvorrichtungen möglich, ohne von der Erfindungsidee abzuweichen, solange eine in Längsrichtung der Vorrichtung bewegliche Sicherungseinrichtung, wie die Hülsenkappe, und ein radial zur Längsachse beweglicher Auslöser für eine Antriebseinrichtung der Verabreichungsvorrichtung vorgesehen sind.

### Bezugszeichen

- 1: Gehäuse
- 2: Ampulle
- 3: Injektionsnadel
- 4: Kolben
- 5: Kolbenstange
- 6: Einstechhülse
- 7: Anschlag
- 8: Anschlag
- 9: erste Feder
- 10: Gehäusewand
- 11: Ausschütthülse
- 12: zweite Feder
- 13: Wand
- 14: Widerstand
- 15: Verlängerung
- 16: Kopfbereich
- 17: Hülsenkappe
- 18: Führungsrille
- 19: Führungsvorsprung
- 20: Auslöserscheibe
- 21: Öffnung
- 22: Öffnung
- 23: Nase
- 24: Nadelschutzhülse
- 25: Feder
- 26: Nadelhülse
- 27: Grundgehäuse
- 28: Sicherungshülse
- 29: Anschlag
- 30: Feder
- 31: Öffnung

## Patentansprüche

1. Vorrichtung zur Verabreichung eines injizierbaren Produkts mit einer Antriebseinrichtung zum Antrieb eines Antriebsgliedes (6; 11) in Richtung einer Längsachse der Vorrichtung, einem Auslöser (20) zum Auslösen der Antriebseinrichtung, einer Sicherungseinrichtung (17), die in einer Sicherungsposition eine Betätigung des Auslösers (20) verhindert, **dadurch gekennzeichnet, dass** die Sicherungseinrichtung (17) entlang der Längsachse der Vorrichtung in eine Entsicherungsposition beweglich ist und der Auslöser (20) in der Entsicherungsposition der Sicherungseinrichtung zum Auslösen radial zur Längsachse beweglich ist, wobei die Sicherungseinrichtung (17) in die Sicherungsposition ohne Betätigung des Auslösers (20) rückstellbar ist.

2. Verabreichungsvorrichtung nach Anspruch 1, **dadurch gekennzeichnet, dass** die Sicherungseinrichtung (17) entgegen einer Ausschüttrichtung des injizierbaren Produkts in die Entsicherungsposition beweglich ist.

3. Verabreichungsvorrichtung nach Anspruch 1 oder 2, **dadurch gekennzeichnet, dass** der Auslöser (20) manuell betätigbar ist.

4. Verabreichungsvorrichtung nach einem der vorhergehenden Ansprüche, **dadurch gekennzeichnet, dass** eine Rastung vorgesehen ist, in der die Sicherungseinrichtung (17) in der Sicherungsposition lösbar einrastet.

5. Verabreichungsvorrichtung nach einem der vorhergehenden Ansprüche, **dadurch gekennzeichnet, dass** der Auslöser (20) rückstellbar ist.

6. Verabreichungsvorrichtung nach einem der vorhergehenden Ansprüche, **dadurch gekennzeichnet, dass** die Antriebseinrichtung eine Einstecheinrichtung (6, 9) zum Einstechen einer Injektionsnadel (3) in eine Oberfläche und/oder eine Ausschütteinrichtung (11; 12) zur Ausschüttung des injizierbaren Produkts aus einem Produktbehälter (2) umfasst.

7. Verabreichungsvorrichtung nach dem vorhergehenden Anspruch, **dadurch gekennzeichnet, dass** die Antriebseinrichtung die Einstecheinnchtung und die Ausschütteinrichtung aufeinander folgend antreibt.

8. Verabreichungsvorrichtung nach einem der vorhergehenden Ansprüche, **dadurch gekennzeichnet, dass** die Sicherungseinrichtung in der Sicherungsposition eine Nadelschutzeinrichtung in einer Schutzposition sichert.

9. Verabreichungsvorrichtung nach einem der vorhergehenden Ansprüche, **dadurch gekennzeichnet, dass** eine Nadelschutzeinrichtung (24) in einer ersten vorgeschobenen Nadelschutzposition den Auslöser (20) sperrt und in einer zweiten zurückgeschobenen Position den Auslöser (20) freigibt.

## Claims

1. A device for administering an injectable product comprising a drive device for driving a drive member (6; 11) in the direction of a longitudinal axis of the device, a release device (20) for releasing the drive device, and a securing device (17) which in a securing position prevents actuation of the release device (20), **characterised in that** the securing device (17) is movable along the longitudinal axis of the device into an unlocked position and the release device (20) is movable radially relative to the longitudinal axis in the unlocked position of the securing device for release purposes, wherein the securing device (17) is resettable into the securing position without actuation of the release device (20).

2. An administration device according to claim 1 **characterised in that** the securing device (17) is movable into the unlocked position in opposite relationship to a dispensing direction of the injectable product.

3. An administration device according to claim 1 or claim 2 **characterised in that** the release device (20) is manually actuable.

4. An administration device according to one of the preceding claims **characterised in that** there is provided a latching means in which the securing device (17) releasably latches in the securing position.

5. An administration device according to one of the preceding claims **characterised in that** the release device (20) is resettable.

6. An administration device according to one of the preceding claims **characterised in that** the drive device includes a piercing device (6, 9) for piercing an injection needle (3) into a surface and/or a dispensing device (11; 12) for dispensing the injectable product from a product container (2).

7. An administration device according to the preceding claim **characterised in that** the drive device successively drives the piercing device and the dispensing device.

8. An administration device according to one of the preceding claims **characterised in that** the securing device in the securing position secures a needle protection means in a protective position.

9. An administration device according to one of the preceding claims **characterised in that** a needle protection means (24) locks the release device (20) in a first advanced needle protection position and releases the release device (20) in a second retracted position.

## Revendications

1. Dispositif d'administration d'un produit injectable équipé d'un dispositif d'entraînement servant à entraîner un élément d'entraînement (6 ; 11) dans la direction d'un axe longitudinal du dispositif, d'un déclencheur (20) servant à déclencher le dispositif d'entraînement, d'un dispositif de sécurité (17) qui empêche, en position de sécurité, l'actionnement du déclencheur (20), **caractérisé en ce que** le dispositif de sécurité (17) est mobile le long de l'axe longitudinal du dispositif, lorsque la sécurité est en position désamorcée, et dans cette position de dispositif de sécurité désamorcée, le déclencheur (20) est mobile pour un déclenchement radial par rapport à l'axe longitudinal, le dispositif de sécurité (17) pouvant être remis en position de sécurité initiale sans actionner le déclencheur (20).

2. Dispositif d'administration selon la revendication 1, **caractérisé en ce que** le dispositif de sécurité (17) est mobile en sens inverse de la direction de déversement du produit injectable, en position désamorcée.

3. Dispositif d'administration selon la revendication 1 ou 2, **caractérisé en ce que** le déclencheur (20) peut être actionné manuellement.

4. Dispositif d'administration selon l'une des revendications précédentes, **caractérisé en ce qu'**une encoche est prévue, dans laquelle le dispositif de sécurité (17) vient s'enclencher de façon détachable en position de sécurité.

5. Dispositif d'administration selon l'une des revendications précédentes, **caractérisé en ce que** le déclencheur (20) peut être remis en position initiale.

6. Dispositif d'administration selon l'une des revendications précédentes, **caractérisé en ce que** le dispositif d'entraînement comprend un dispositif de piqûre (6, 9) servant à piquer une aiguille d'injection (3) dans une surface et/ou un dispositif de déversement (11 ; 12) servant à déverser le produit injectable hors d'un récipient à produit (2).

7. Dispositif d'administration selon la revendication précédente, **caractérisé en ce que** le dispositif d'entraînement entraîne successivement le dispositif de piqûre et le dispositif de déversement.

8. Dispositif d'administration selon l'une des revendications précédentes, **caractérisé en ce que** le dispositif de sécurité en position de sécurité sécurise un dispositif de protection d'aiguille dans une position de protection.

9. Dispositif d'administration selon l'une des revendications précédentes, **caractérisé en ce qu'**un dispositif de protection d'aiguille (24) bloque le déclencheur (20) dans une première position de protection d'aiguille avancée et relâche le déclencheur (20) dans une deuxième position rétractée.
